# EUROPEAN PATENT APPLICATION

(11) **EP 1 589 112 A1**
(43) Date of publication of application: **26.10.2005**
(21) Application number: 04076209.8
(22) Date of filing: 21.04.2004
(51) Int. Cl.: C12P 7/22, C12R 1/40

(54) **Microbial production of aromatic acids**

(71) Applicant: Nederlandse Organisatie voor Toegepast-Natuurwetenschappelijk Onderzoek TNO, 2628 VK Delft (NL)
(72) Inventor: Wery, Jan, 7213 TD Gorssel (NL)
(74) Representative: Winckels, Johannes Hubertus F.

(57) **Abstract**

The invention relates to the enzymatic production of aromatic acids using renewable carbon sources, such as sugars. Provided is a method for the microbial production of an aromatic acid from a fermentable carbon substrate using a host cell capable of producing said aromatic acid, for instance cinnamic acid, para-hydroxycinnamic acid (PHCA), and comprising an efflux pump for said aromatic acid. A preferred host cell comprises a member of the proton-dependent resistance/nodulation/cell division (RND) family of efflux pumps, preferably the solvent resistance pump srpABC of *P. putida* strain S12. Also provided is a method for providing a host cell that overproduces an aromatic acid, comprising subjecting a population of host cells to random insertional mutagenesis, screening said mutated host cells for aromatic acid production and identifying at least one host cell that overproduces said aromatic acid compared to a parent host cell that has not been subjected to random mutagenesis.

## Description

The invention relates to the production of aromatic acids using renewable carbon sources, such as sugars. More specifically, it relates to an improved method for the microbial production of aromatic acids using a host cell comprising an enzymatic pump capable of actively secreting said aromatic acid out of said host cell, for example into the culture medium. Whereas the invention will be mainly described with reference to the production of cinnamic acid and para-hydroxy cinnamic acid, a method of the invention is also advantageously used for the microbial production of other aromatic acids.

The chemical synthesis of aromatic acids is based on non-renewable carbon substrates and is thus undesirable from an environmental point of view (e.g. CO₂ emission). Furthermore, the chemical synthesis of aromatic acids is often laborious. Therefore, large scale synthesis of aromatic acids is preferably performed using a microbial production system and renewable carbon sources, such as sugars.

Examples of commercially important aromatic acids include cinnamic acid (also known as cinnamate) and *para*-hydroxy cinnamic acid (PHCA; also known as p-coumarate). PHCA is a useful monomer for the production of Liquid Crystal Polymers (LCP). LCP's may be used in electronic connectors and telecommunication and aerospace applications. LCP resistance to sterilizing radiation has also enabled these materials to be used in medical devices as well as chemical, and food packaging applications. Furthermore, PHCA can be used in sun screen products and cosmetics and as antioxidant in food stuff. An important pharmaceutical for high blood pressure and stroke prevention, known as coumarin or oxy-cinnamic acid, is a derivative of cinnamic acid. For an overview of the occurrence and metabolism of cinnamic acid and related compounds see a Review by J.A. Hoskins (1984; Journal of Applied Toxicology, 4:283-292).

Cinnamic acid and PHCA both occur naturally in plants, where they serve as intermediates in the lignin biosynthetic pathway in plants (Plant Biochemistry, Ed. P. M. Dey, Academic Press, 1997). Methods of isolation and purification of cinnamic acid and PHCA from plants are known (R. Benrief, *et al*., Phytochemistry, 1998, 47,825-832; WO 97/2134). However, these methods are time consuming and cumbersome and a more facile method of production is needed for the commercial, large scale synthesis of these aromatic acids.

The microbial production of cinnamic acid and PHCA using a sugar fermentation route is known from WO 02/090523, wherein a microbial host cell is engineered with key enzymes involved in aromatic acid synthesis such that it produces the desired aromatic acid(s). However, further optimization and improvement of the product yield of known microbial production methods is hampered by the fact that aromatic acids, once produced, typically accumulate in the host cell. As a result, the accumulated product inhibits one or more enzymes involved in its production such that a further increase in aromatic acid synthesis is restrained. This phenomenon is also referred to as a negative feedback control mechanism.

The invention now provides the insight that a host cell comprising an efflux pump that is capable of actively transporting an aromatic acid out of the cell is advantageously used for the microbial production of aromatic acids.

Provided is a method for the microbial production of an aromatic acid from a fermentable carbon substrate using a host cell capable of producing said aromatic acid and comprising an efflux pump for said aromatic acid. In a method of the invention, the host cell can secrete the aromatic acid into the culture medium, such that product accumulation in the cell and, conceivably, negative feedback, is minimized. As a result, higher product yields can be achieved compared to existing microbial production systems that employ host cells which cannot effectively secrete the aromatic acid produced. In addition, a method of the invention does not require the harvest and further processing of host cells to obtain the desired end product. Instead, the culture medium of the host cell enriched with the end product can be taken and subjected to further processing to isolate and/or purify the product. Thus, a method is provided simple in its use that can be performed in a continuous fashion, whereas a batch wise cultivation is possible as well.

Suitable host cells for use in a method of the invention include microbial cells which can produce an aromatic acid and which display a resistant phenotype towards hydrophobic solvents such as toluene and octanol. However, also (bacterial) host cells which are not solvent-resistant but which do comprise an efflux pump capable of exporting aromatic acids are of use in the present invention.

Many different mechanisms have been described that contribute to solvent resistance, one of which relates to an energy-dependent efflux pump which actively keeps toxic solvents out of the interior of the cell. Solvent resistant or tolerant host cells are advantageously used in a method of the invention because the pump conferring resistance or tolerance towards organic solvents has been shown to possess a very broad specificity, taking organic compounds that by virtue of their chemico-physical characteristics accumulate into the bacterial membrane such as aromatics, alcohols, alkanes etc. as a substrate (Kieboom et al. 1998. J. Biol. Chem. 273:85-91). Undissociated aromatic compounds will by virtue of similar chemico-physical characteristics also partition effectively to the cell membrane where they act as a substrate of such a pump.

In one embodiment of the invention, a host cell, preferably a Gram-negative bacterium, comprises a member of the proton-dependent resistance/nodulation/cell division (RND) family of efflux pumps. RND-type efflux pumps belong to the multidrug resistance (MDR) pumps. They have an extremely broad substrate specificity and protect bacterial cells from the actions of antibiotics on both sides of the cytoplasmic membrane. Members of this family have been shown to be involved in export of antibiotics, metals, and oligosaccharides involved in nodulation signaling. RND-type efflux pumps usually function as three-component assemblies spanning the outer and cytoplasmic membranes and the periplasmic space of Gram-negative bacteria. Examples of suitable RND-type efflux pumps for use in a method of the invention can be found in Tseng, T.T., Gratwick, K.S., Kollman, J., Park., D., Nies, D.H., Goffeau, A., & Saier Jr., M.H. (1999) The RND permease superfamily: an ancient, ubiquitous and diverse family that includes human disease and development proteins. J. Mol. Microbiol. Biotechnol. 1: 107-125.

In one embodiment, a method provided herein for the production of an aromatic acid uses a host cell comprising a solvent resistance pump, preferably the solvent resistance pump srpABC of *P. putida* S12 (Isken et al. 1996 J. Bacteriol. 178:6056; Kieboom et al. 1998. J. Biol. Chem. 273:85-91). The deduced amino acid sequences of the proteins encoded by the srpABC genes have extensive homology with those of the RND family of efflux pumps. It is composed of three protein components that together span the inner and outer membranes of Gram-negative bacteria: an inner membrane transporter (SrpB analogues), an outer membrane channel (SrpC analogues), and a periplasmic linker protein (SrpA analogues). Dendrograms showing the phylogenetic relationship of SrpA, SrpB, and SrpC to other proteins involved in multidrug resistance are shown in Kieboom et al. 1998 J. Biol. Chem. 273:85-91. The srpABC-encoded proteins show the most homology with those for the mexAB/oprM-encoded multidrug resistance pump found in *Pseudomonas aeruginosa*. SrpA, SrpB, and SrpC are 57.8, 64.4, and 58.5% identical to MexA, MexB, and OprM, respectively. In one embodiment of the present invention, a host cell comprises an efflux pump consisting of an inner membrane transporter, an outer membrane channel, and a periplasmic linker protein belonging to the RND-family of efflux pumps wherein the proteins show a homology of at least 50%, preferably at least 55% to the SrpA, SrpB or SrpC proteins of *P*. *putida* S12. In fact, any functional equivalent of known solvent efflux pumps that can use an aromatic acid as a substrate are suitably used in a method of the invention.

In a preferred embodiment, a method of the invention uses a *Pseudomonas* spp., preferably *Pseudomonas putida*, more preferably *P*. *putida* strain S 12 as a host cell for the production of an aromatic acid.

In one embodiment, a method is provided for the microbial production of cinnamic acid and PHCA using a host cell that can convert the fermentable carbon substrate into an aromatic amino acid (phenylalanine or tyrosine), which is subsequently converted into the aromatic acids cinnamic acid and PHCA, respectively. Once produced, cinnamic acid and PHCA are actively transported out of the host cell by an efflux pump, preferably by a member of the proton-dependent resistance/nodulation/cell division (RND) family of efflux pumps, more preferably srpABC.

Phenylalanine and tyrosine, which in a method of the invention can serve as precursors for aromatic acids, are naturally present in micro-organisms. However, for an optimal synthesis of aromatic acids a host cell preferably over-produces one or more aromatic acid precursors (e.g. aromatic amino acids) such that the substrate level does not limit aromatic acid production by the host cell. Methods to increase aromatic amino acid synthesis in a micro-organism are known in the art. In one embodiment, a host cell is selected for increased resistance against a toxic analog of an aromatic amino acid. For example, mutant micro-organisms can be selected for resistance against toxic (fluoro-)analogs of phenylalanine and tyrosine. These insensitive mutants often appear to produce high levels of phenylalanine and tyrosine (GB 1071935; US3,709,785).

It is also possible to obtain a recombinant host cell with increased phenylalanine and tyrosine production by overexpression of one or more key genes in the biosynthesis of phenylalanine and/or tyrosine (Ikeda 2003. Amino acid production processes. P.1-35. in T. Scheper (Ed.), Advances in Biochemical Engineering/Biotechnology, Vol. 79. Springer-Verlag, Berlin Heidelberg).

A recombinant microorganism with increased phenylalanine and tyrosine production is advantageously used as a host cell in a method for the microbial production of cinnamic acid and PHCA. Phenylalanine is enzymatically converted into cinnamic acid through the action of phenylalanine ammonia lyase (PAL; EC 4.3.1.5). Therefore, a host cell of the invention preferably comprises, in addition to an efflux pump, at least one gene encoding PAL activity. The term "PAL activity" refers to the ability of a protein to catalyze the conversion of phenylalanine to cinnamic acid.

In plants, cinnamic acid is subsequently converted to PHCA by cinnamate-4-hydroxylase (C4H), a cytochrome P450-dependent monooxygenase (P450). Thus, it is evident that one possible route to PHCA is via phenylalanine ammonia lyase (PAL) from phenylalanine. However this route also requires the presence of the second enzyme, cinnamate-4-hydroxylase, an enzyme which is rare in most micro-organisms. Information available indicates that PAL from some plants and micro-organisms can accept tyrosine as substrate in addition to its ability to convert phenylalanine to cinnamate. In such reactions the enzyme activity is designated tyrosine ammonia lyase (TAL). Conversion of tyrosine by TAL results in the direct formation of PHCA from tyrosine without the intermediacy of cinnamate. Therefore, in one embodiment of the invention a host cell capable of producing cinnamic acid and PHCA is used which comprises "PAL/TAL activity", which refers to a protein which contains both PAL and TAL activity. Such a protein has at least some specificity for both tyrosine and phenylalanine as an enzymatic substrate.

However, all natural PAL/TAL enzymes prefer to use phenylalanine rather than tyrosine as their substrate. The level of TAL activity is always lower than PAL activity, but the magnitude of this difference varies over a wide range. Exception to this rule are the PAL/TAL enzymes from *Rhodotorula* spp. and from *Rhodosporium toruloides* (US4,636,466; Hanson and Havir in The Biochemistry of Plants ; Academic: New York, 1981; Vol. 7, pp 577-625), in which a ratio of TAL catalytic activity to PAL catalytic activity is approximately 0.58. For that reason, a host cell is preferably provided with a PAL/TAL gene from *Rhodotorula* spp. or *Rhodosporium toruloides*. Alternatively, a host cell comprises a "modified PAL/TAL" activity which refers to a protein which has been derived from a wild type PAL enzyme but which has been genetically engineered such that the TAL activity is greater than PAL activity. As such, a modified PAL/TAL protein has a greater substrate specificity for tyrosine than for phenylalanine. Directions to obtain a modified PAL/TAL protein can be found in WO02/090523.

As said, the invention also provides a method for the microbial production of aromatic acids other than cinnamic acid and derivatives thereof. For example, a host cell may be genetically engineered with one or more foreign enzyme activities such that it produces an aromatic acid that is otherwise not produced in said host cell. In one embodiment, a host cell A is provided with the enzyme B from organism C to obtain a host cell comprising an efflux pump that is capable of producing aromatic acid D. Next to cinnamic acid and PHCA also benzoate and parahydroxybenzoate can be aromatic acids which can be produced in host cells.

Recombinant host cells can be obtained using methods known in the art for providing cells with recombinant nucleic acids. These include transformation, transconjugation, transfection or electroporation of a host cell with a suitable plasmid (also referred to as vector) comprising the nucleic acid construct of interest operationally coupled to a promoter sequence to drive expression. Typically, the plasmid also comprises a selection marker which confers the host cell with resistance to a selective agent, such as an antibiotic. Culturing the host cell in the presence of the selective agent, i.e. under a selective pressure, ensures that the plasmid is maintained by the host cell. However, selective agents are generally expensive. Especially in large scale microbial production systems, it is preferred to culture under the cheapest condition possible, i.e. without using selective pressure. In addition, it can be encountered that the host cell looses the extrachromosomal plasmid without loosing resistance against the selective agent. Therefore, a host cell for use in a method of the invention is preferably genetically modified using a procedure that does not rely on culturing under selection pressure and which results in a genetically stable recombinant host cell. In one embodiment, a host cell is provided with a nucleic acid of interest, for example a gene encoding an enzyme involved in aromatic acid synthesis such as PAL, using insertional mutagenesis. Herein, an isolated nucleic acid is inserted into the genome of the host cell. Insertion can be site-directed or random. In a preferred embodiment, the invention provides a method for providing a host cell (over)producing an aromatic acid comprising insertional mutagenesis. Insertional mutagenesis advantageously makes use of a transposon or a plasposon. A plasposon is a mini-transposon with an origin of replication (see Dennis and Zylstra, 1998). In a more preferred embodiment, random insertional mutagenesis is used to provide a genetically modified variant host cell for use in a method of the invention. For example, a collection of variant host cells, preferably *P. putida*, is provided which each contain a mini-transposon comprising the PAL gene randomly inserted into their genomic DNA. In each variant host cell, the genomic DNA will contain a mutation at the site of integration of the transposon. In a some cases, a mutation can lead to the inactivation of a genetic element (e.g. a coding region or a regulatory element) that is involved in aromatic acid metabolism in said host cell. For example, an enzyme may become inactivated which normally degrades or further metabolises the desired aromatic acid produced by the host cell. This will of course contribute to an increased aromatic acid yield. Alternatively, a metabolic side-route is inactivated due to the insertional mutagenesis procedure such that the metabolic flux of precursor(s) into the biosynthesis of the aromatic acid is increased.

Thus, in one aspect, the invention provides a method for providing a host cell that overproduces an aromatic acid, comprising subjecting a population of host cells to random insertional mutagenesis, screening said mutated host cells (also referred to as 'variant' host cells) for optimized aromatic acid production and identifying at least one mutated host cell that overproduces said aromatic acid compared to a parent host cell that has not been subjected to random mutagenesis. Specifically such a method comprises selecting of a host cell for increased accumulation of phenylalanine and/or tyrosine by screening for mutants resistant against toxic analogs of an aromatic amino acid.

Variant host cells in which insertional mutagenesis has resulted in a useful mutant can be readily identified by screening for increased production levels of the desired compound. Since a host cell of the invention can secrete the aromatic acid into the medium, aliquots of the culture medium used for growing a variant host cell can be easily analysed for the presence of increased aromatic acid production. For example, example 3 describes the screening of a collection of variant host cells obtained using random insertional mutagenesis for increased production of cinnamic acid and PHCA. Also provided is a variant host cell obtainable by such a method. Of particular interest are variant host cells wherein at least one enzyme involved in the degradation of said aromatic acid is disrupted or wherein a metabolic side-route of the biosynthesis of said aromatic acid is disrupted.

A host cell according to the invention is cultured in a culture medium comprising a fermentable carbon substrate according to standard microbial culturing conditions. The term "fermentable carbon substrate" refers to a carbon source capable of being metabolized by the host cell and particularly to carbon sources selected from the group consisting of monosaccharides, oligosaccharides, polysaccharides, and one-carbon substrates or mixtures thereof. A preferred carbon substrate in a method of the invention for the production of an aromatic acid is glucose.

Furthermore, the invention provides use of a host cell comprising an efflux pump for an aromatic acid, preferably a member of the proton-dependent resistance/nodulation/cell division (RND) family of efflux pumps, more preferably the solvent resistance pump srpABC of *P. putida* strain S12, for the microbial production of an aromatic acid. The host cell may be genetically modified to (over)produce the aromatic acid, for example by overexpression of at least one key enzyme in the aromatic acid biosynthesis. In a preferred embodiment, the host cell used is a *Pseudomonas* spp., preferably *Pseudomonas putida*, more preferably *P. putida* strain S12. In another preferred embodiment, said host cell is used for the production of cinnamic acid or para-hydroxycinnamic acid (PHCA).

The invention is further illustrated by the examples below.

### LEGENDS

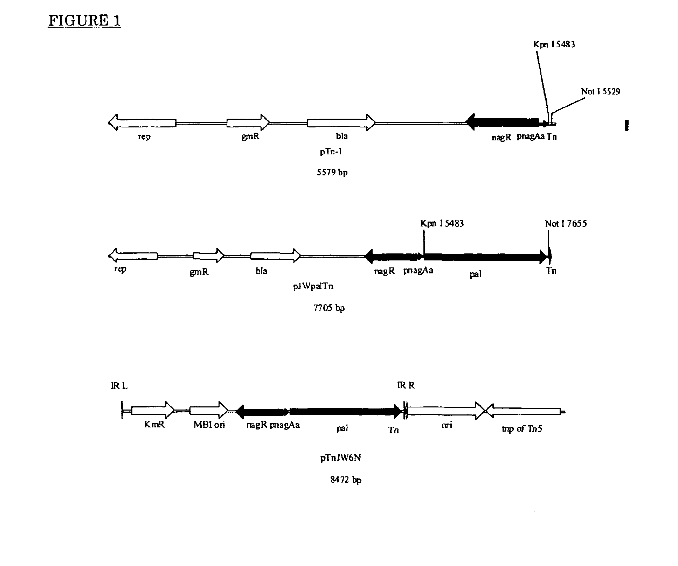

### Figure 1. Schematic representation of the DNA constructs pTn-1, pJWpalTn and pTnJW6N described in Examples 1 and 2.

Abbreviations used: *nagR*/*PnagAa*, regulatory DNA sequence for naphthalene degradation from *Comomonas testosteroni;* rep, codes for the replication function; GmR, gentamycin resistance marker; bla, ampicillin resistance marker; Tn, transcription terminator; bp, basepairs; IR (L/R), Left/Right inverted repeat sequence of transposon Tn5; tnp, transponase gene of transposon 5.

### EXAMPLES

### Example 1: Cloning of a nucleic acid sequence encoding phenylalanine ammonia lyase from R. toruloides into E.coli - P.putida expression plasmids and transformation into E. coli and P. putida S12.

DNA encoding phenylalanine ammonia lyase (the *pal* gene; GenBank accession number X51513) was amplified using PCR from a cDNA collection obtained from *R. toruloides* mRNA as described by Sarkissian et al. (1999. Proc. Natl. Acad. Sci. USA 2:96) using oligonucleotides designed for the 5'- and 3'- end of the *pal* DNA. The oligonucleotide homologous to the 5'-end of *pal* (oligo 1) contains an additional *Kpn*I restriction site and the oligonucleotide homologous to the 3'-end (oligo 2) an additional *Not*I restriction site (see Table 1).

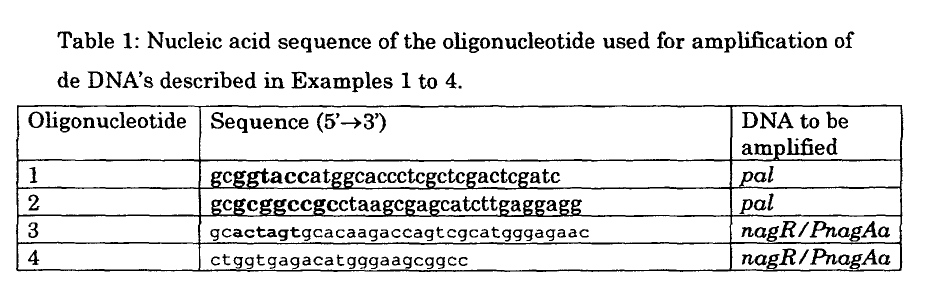

The PCR-amplified *pal* gene was digested with *Kpn*I and *Not*I and ligated into the plasmid pTn-1 (Fig 1.) which had also been digested with *Kpn*I and *Not*I. This resulted in the DNA construct pJWpalTn (Fig. 1) wherein the pal gene was placed under the control of the inducible regulatory sequence *nagR*/*PnagAa* (Hüsken et al. 2001. Appl. Microbiol. Biotechnol. 55:571-577). Hereinafter, this configuration is referred to as the *nagR*/ *PnagAa::pal* cassette.

PJWpalTn was introduced into *E.coli* and *P*. *putida* S12 host cells by standard transformation procedures. The *P.putida* S12 host cell genetically engineered to express phenylalanine ammonia lyase is referred to as S12pal. The host cells were cultured under selection pressure (ampicillin for *E.coli* and gentamycin for *P.putida* S12) to ensure that the host cells maintained the plasmid.

### Example 2: Isolation of a collection of P. putida S12 variants.

This example describes the insertion of the *nagR*/*PnagAa::pal* cassette into the chromosomal DNA of *P. putida* S12 to generate *P. putida* S12 variants which can be cultured in the absence of selection pressure (e.g. an antibiotic) without loosing the *pal* gene. To this end, the *nagR*/*PnagAa::pal* cassette was cloned in the mini-transposon section of the plasposon pTnMod-KmO (Dennis and Zylstra. 1998 Appl. Environ. Microbiol. 64:2710-2715) by PCR amplification of the nagR/PnagAa DNA sequence from pTn-1 using oligonucleotides 3 and 4 shown in Table 1. The oligonucleotide homologous to the 5'-end (oligo 3) contains an *Spe*I restriction site. The amplified DNA was digested with *Avr*II and *Spe*I and ligated into the *Spe*I-digested plasposon pTnMod-KmO. The resulting DNA construct was partially digested with Bsp120I and KpnI and ligated to the *pal* gene removed from the plasmid pJWpalTn using *Kpn*I/*Not*I. The DNA construct obtained, pTnJW6N (Fig. 2) was transferred to *P. putida* S12 via triparental mating (Dennis and Zylstra. 1998 Appl. Environ. Microbiol. 64:2710-2715). As a result of this approach, a collection of more than 18.000 genetically modified *P. putida* S12 variant host cells were obtained wherein the mini-transposon part of pTnJW6N, including the *nagR*/*PnagAa::pal* cassette, is randomly inserted into the chromosomal DNA. Because of the random integration, these variants are also randomly genetically modified at the site of integration. It was expected that the random mutagenesis would, in at least some of the variants, have a positive effect on the production of an aromatic acid, for example through inactivation of enzymes involved in the breakdown of a precursor, e.g. phenylalanine or tyrosine, or through an increase in the metabolic flux to these aromatic amino acids by disturbing the side-routes of this flux.

### Example 3: Screening of P. putida S12 variants for increased production of cinnamic acid and PHCA.

18000 variant *P. putida* S12 host cells of Example 2 were tested for their ability to overproduce cinnamic acid and PHCA in comparison to the *P. putida* S12 parent strain. The optical density at 278 nm and 310 nm of the culture medium of the different variants was determined as a relative measure for cinnamic acid and PHCA, respectively. Five variants were selected which exhibited an increased production. The variants were genetically analysed for the site of integration of the *nagR*/*PnagAa::pal* cassette and stable passing of the cassette to at least 100 further generations in the absence of a selection pressure was confirmed. One variant, S12pal1, was tested as above for the production of cinnamic acid and PHCA in more detail during growth.

### Example 4: Production of cinnamic acid and PHCA by the genetically modified P.putida S12 variant host cells S12pal and S12 pall.

To determine the production of cinnamic acid and PHCA by the genetically modified *P.putida* S12 variant host cells S12pa1 and S12 pall, these host cells were cultured in an aqueous mineral medium (Hartmans et al. 1989. Appl. Environ. Microbiol. 55:2850-2855) supplemented with 20 mM glucose and 0.1 mM sodium salicylate in case induction of expression of the *pal* gene into a functional phenylalanine ammonia lyase was required. As a control for the source of phenylalanine ammonia lyase activity in S12pal and S12 pall, we also tested a *P.putida* variant provided with the pTn-1 plasmid without the *pal* gene. Except for S12pal1 cultures, 10 mg/L gentamicine was added to the culture medium. All variant host cells were cultured in the presence or absence of 1 mM exogenous phenylalanine or tyrosine. The levels of cinnamic acid and PHCA as well as the total cellular protein content in the cultures were determined at various time points using High Performance Liquid Chromatography (HPLC).
In addition, at various time points cell free extracts were prepared from the cultures which were analysed for protein content and phenylalanine ammonia lyase activity. Hereto, the formation of cinnamic acid and PHCA was determined using HPLC at various time points following the addition of 1 mM phenylalanine or 1 mM tyrosine to the cell free extracts.

The results of these measurements were used to determine the following parameters for cinnamic acid and PHCA production (see Table 2):
- the maximal concentration of cinnamic acid and PHCA in the culture medium or the cell free extract
- the maximal specific rate of cinnamic acid and PHCA production in the culture medium or the cell free extract.

**Table 2:**

| Production of cinnamic acid and PHCA in cultures and cell-free extracts of *P.putida* S12 variants genetically modified with the gene encoding Phenylalanine ammonia lyase (PAL). | | | | |
|---|---|---|---|---|
| Cultures of CVE ^{a)} | Maximal concentration^{b)} (µM) | | Maximal specific production rate^{c)} (nmol.min⁻¹.mg⁻¹) | |
| | Cinnamic acid | *Para-* hydroxycinnamic acid | cinnamic acid | *Para-* hydroxycinnamic acid |
| S12 control | 0 | 0 | 0 | 0 |
| S12pal | 101 | 152 | 1.0 | 1.2 |
| S12pal1 | >101 | >152 | >1.0 | >1.2 |
| S12 control + fen. | 0 | 0 | 0 | 0 |
| S12pal + fen. | 267 | 134 | 3.5 | 1.1 |
| S12pal1 + fen. | nd | nd | nd | nd |
| S12 control + tyr. | 0 | 0 | 0 | 0 |
| S12pal + tyr. | 149 | 207 | nd | 2.5 |
| S12pal1 + tyr. | nd | nd | nd | nd |
| CVE S12 control + fen. | 0 | 0 | 0 | 0 |
| CVE S12pal + fen. | nd | nd | 17.2 | nd |
| CVE S12pal1 + fen. | nd | nd | nd | nd |
| CVE S12 control + tyr. | 0 | 0 | 0 | 0 |
| CVE S12pal + tyr. | nd | nd | nd | 3.8 |
| CVE S12pall+ tyr | nd | nd | nd | nd |

| | | | | |
|---|---|---|---|---|
| a) CVE, cell free extract; S12 control, *P. putida* S12 with vector pTn-1; S12pal, *P. putida* S12 with construct pJWpalTn; S12pal1, *P. putida* S12 with *nagR*/*PnagAa*:*pal* cassette stably integrated in the chromosome; + fen., with 1mM phenylalanine added; + tyr, with 1mM tyrosine added. | | | | |
| b) The maximal concentration cinnamic acid en *para*-hydroxycinnamic acid in the culture medium or CVE. c) The maximal specific production speed is defined as the maximal amount of cinnamic acid or *para*-hydroxycinnamic acid (in millimol) that accumulates per minuet per gram cell protein in the culture medium or CVE. Nd = not determined. | | | | |

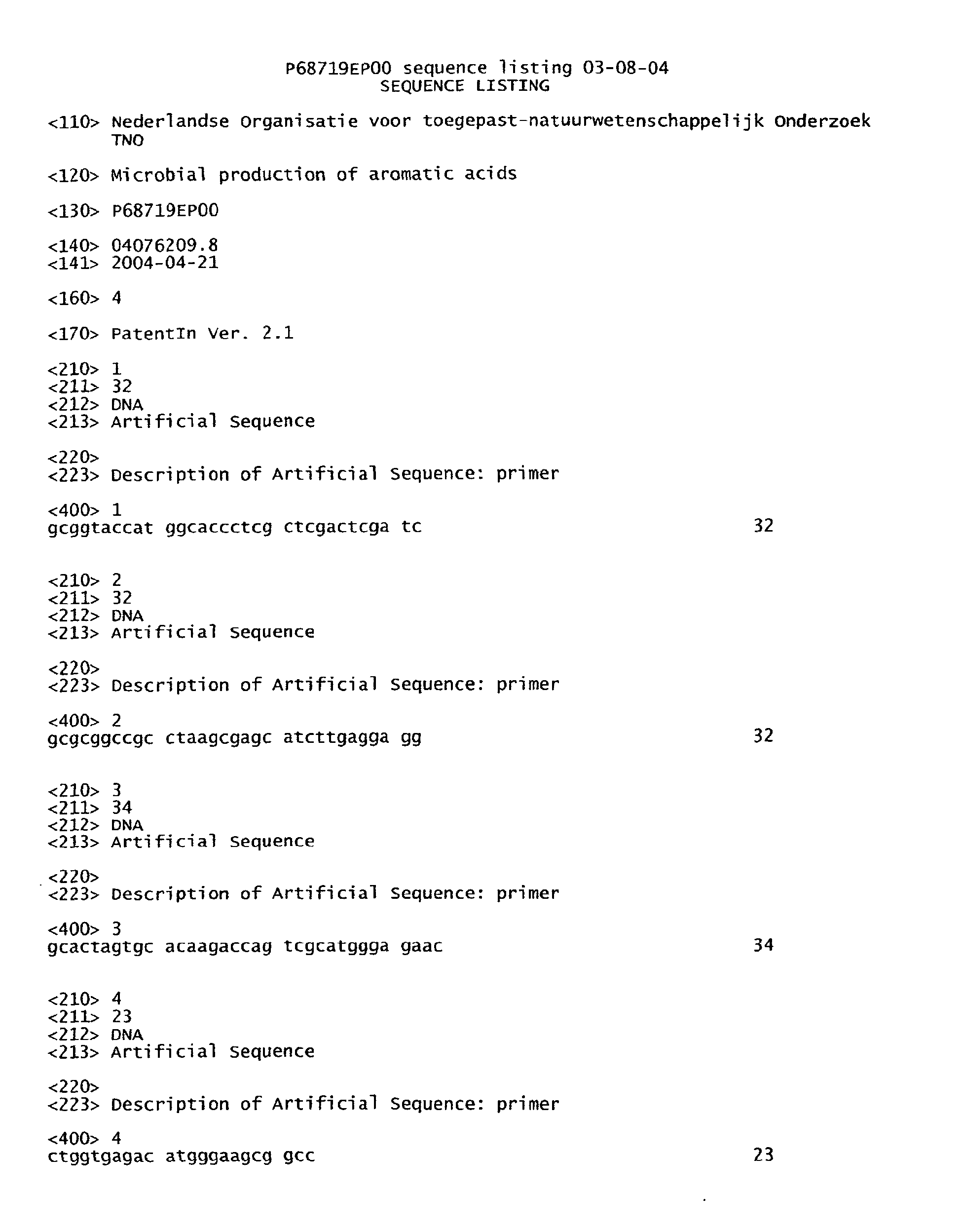

## Claims

1. A method for the enzymatic production of an aromatic acid in a microbial host cell using a fermentable carbon substrate, wherein said host cell comprises an efflux pump for said aromatic acid.

2. A method according to claim 1, wherein said efflux pump is a member of the proton-dependent resistance/nodulation/cell division (RND) family of efflux pumps, preferably a solvent resistance pump, more preferably the solvent resistance pump srpABC of *P. putida* strain S12.

3. A method according to claim 1 or 2, wherein said host cell is a *Pseudomonas* spp., preferably *Pseudomonas putida,* more preferably P. putida strain S12.

4. A method according to any one of claim 1 to 3, wherein said host cell expresses or overexpresses at least one enzyme involved in the biosynthesis of said aromatic acid.

5. A method according to any one of claims 1 to 4, wherein said host cell is genetically modified to produce or overproduce said aromatic acid or a precursor thereof.

6. A method according to claim 4 or 5, wherein said host cell is genetically modified using insertional mutagenesis, preferably random insertional mutagenesis, more preferably using a transposon construct.

7. A method according to any one of claims 1 to 6, wherein said aromatic acid is selected from the group consisting of cinnamic acid, para-hydroxycinnamic acid (PHCA), para-hydroxybenzoate, and benzoate.

8. A method according to claim 7, wherein said host cell overexpresses phenylalanine ammonia lyase (PAL), preferably PAL with tyrosine ammonia lyase (TAL) activity, and /or phenylalanine hydroxylase (PAH).

9. A method according to any one of claims 1 to 8, wherein said host cell is selected for increased accumulation of phenylalanine and/or tyrosine by screening for mutants resistant against a toxic analog of an aromatic amino acid.

10. A method according to any one of claims 1 to 9, wherein said carbon substrate is selected from the group consisting of monosaccharides, oligosaccharides, polysaccharides, carbon dioxide, methanol, formaldehyde, formate, and carbon-containing amines, preferably glucose.

11. A method for providing a host cell that overproduces an aromatic acid, comprising subjecting a population of host cells to random insertional mutagenesis, screening said mutated host cells for aromatic acid production and identifying at least one mutated host cell that overproduces said aromatic acid compared to a parent host cell that has not been subjected to random mutagenesis.

12. A host cell obtainable by a method according to claim 10.

13. A host cell according to claim 11, wherein at least one enzyme involved in the degradation of said aromatic acid is disrupted or wherein a metabolic side-route of the biosynthesis of said aromatic acid is disrupted.

14. Use of a host cell comprising an efflux pump, preferably a member of the proton-dependent resistance/nodulation/cell division (RND) family of efflux pumps, more preferably the solvent resistance pump srpABC of *P. putida* strain S 12, for the microbial production of an aromatic acid.

15. Use according to claim 13, wherein said host cell is a *Pseudomonas* spp., preferably *Pseudomonas putida*, more preferably *P. putida* strain S12.

16. Use according to claim 13 or 14, wherein said aromatic acid is cinnamic acid or para-hydroxycinnamic acid (PHCA).
